Europäisches Patentamt

European Patent Office

Office européen des brevets

(11) Publication number: **0 056 855**
**B1**

(12) EUROPEAN PATENT SPECIFICATION

(45) Date of publication of patent specification: **21.11.85**

(21) Application number: **81109961.3**

(22) Date of filing: **27.11.81**

(51) Int. Cl.⁴: **A 61 K 9/00,** A 61 L 2/00,
B 01 D 13/00, C 02 F 9/00

(54) **Preparation of infusion grade water.**

(30) Priority: **02.12.80 US 212142**

(43) Date of publication of application:
**04.08.82 Bulletin 82/31**

(45) Publication of the grant of the patent:
**21.11.85 Bulletin 85/47**

(84) Designated Contracting States:
**BE CH DE FR GB IT LI NL SE**

(56) References cited:
DE-A-2 919 315
FR-A-2 126 970
FR-A-2 309 238
GB-A-2 002 736
GB-A-2 020 997
US-A-3 774 763
US-A-3 836 458
US-A-4 072 610
US-A-4 181 694
US-A-4 265 760
US-A-4 267 053

(73) Proprietor: **TRACOR, INC.**
**6500 Tracor Lane**
**Austin, TX 78721 (US)**

(72) Inventor: **Klein, Elias**
**4430 St. Bernard Avenue**
**New Orleans Lousiana 70122 (US)**
Inventor: **Beach, Douglas J.**
**1616 Keogh Street**
**Burglington North Carolina 27215 (US)**

(74) Representative: **Jack, Bruce James et al**
**FORRESTER & BOEHMERT Widenmayerstrasse**
**4/I**
**D-8000 Munchen 22 (DE)**

Courier Press, Leamington Spa, England.

## Description

The invention relates to a portable system for preparing infusion grade water.

It is known that in the preparation of parenteral solutions, the starting water must be of a high purity. Parenteral solutions must be prepared without either chemical or biological contaminants.

The basic requirements for infusion grade solutions are that they be bacteria free, have a base of chemically pure water, are endotoxin free and monitored in a strict quality control system. The main concern in parenteral solutions is the presence of pyrogens. A pyrogen is defined as any chemical or biological substance that will cause a rise in temperature, that is, a febrile response when intravenously or intramuscularly introduced into a subject mammal. It has been found that endotoxins of gram-negative bacteria are pyrogenic and are the most common cause of septic shock.

Bacterial endotoxins are lipopolysaccharides derived from the cell wall of the gram-negative bacteria. They are polymeric materials having molecular weights between 400,000 and 4,000,000 Daltons. The polymer can be depolymerized to basic units of 10,000 to 20,000 Daltons by being treated with strong detergents or bile salts. However, in pure water depolymerization would not occur.

Pyrogenic reactions to endotoxins can be regularly elicited in man, rabbits and certain other mammals. In minimal dosages endotoxin will induce an elevation from the normal temperature of the recipient. Acute endotoxin poisoning can lead to irreversible shock and death.

Endotoxin is shed from the outer surface of gram-negative bacteria. It is destroyed by a heat treatment of 175°C for a period of approximately two hours. Endotoxin is not affected by sterilization via autoclaving.

The most widely used method for pyrogen removal is distillation. In this method large quantities of water are distilled, stored and then packaged in a sterile environment for delivery to hospitals and emergency units. Various chemical treatments have also been proposed to remove pyrogen from water and parenteral preparations. The main difficulty in the chemical treatment lies in the removal of the chemical and its reaction products after the completion of the treatment process.

Removal of endotoxin by any means other than distillation was not practical prior to the development of ultrafiltration membranes. Ultrafilters allow selection in a dissolved species. Since endotoxins of the gram-negative bacteria have a high moleculur weight ultra-filters may be used to remove these pyrogenic endotoxins.

While being the most effective historical method for producing pyrogen free water, distillation is in terms of resources the most costly system available. Further, distillation systems do not lend themselves to practical utility in military field hospitals and other emergency field medical units. Military medical units require large volumes of sterile, nonpyrogenic fluids for infusion. Distillation techniques do not lend themselves to on site production on an as needed basis. Using the distillation methods requires that large inventories of sterile non-pyrogenic water be transported to the location.

With quality control and maintained sterility being a primary factor in the overall preparation and storage of sterile nonpyrogenic infusion grade water, endotoxin testing is also a constraint with regard to on-site units. Historically, a rabbit test was used to determine the presence of pyrogenic endotoxins. The rabbit test for pyrogens requires injection of 10 ml of test solution into the ear veins of three rabbits weighing not less than 1.5 kg each. The rabbits must be individually caged in a shock-free environment, and their temperature must be taken 40 minutes prior to injection and for three hours after injection. If no one rabbit shows a temperature rise of 0.6°C or more, and if the collective increases in temperature totals no more than 1.4°C, the test solution is determined to be nonpyrogenic. The disadvantages of the rabbit test include maintenance of the test animals, a minimum of three hours for performance, the requirement of three rabbits per test, and the requirement of skilled personnel to administer and record the temperature changes.

With the advent of a simpler *in vitro* test that requires only a one hour incubation time and uses less than 1 ml of test solution the potential for qualifying field unit devices for preparation of infusion grade water increased. The test is an enzymatic reaction that occurs when certain clottable proteins found in circulating amebocytes encounter endotoxin. This test uses a Limulus Amebocyte Lysate and is referred to as LAL. Because LAL is a reliable, sensitive, and rapid *in vitro* test that can be performed with a minimum of equipment, a minimum space requirement and no unusual skills or personnel, it is a primary choice for use in an on-site field system for generation of infusion grade water.

A major problem in practical generation of infusion grade water on site lies in retaining the nonpyrogenic characteristic of the water. In conjunction with this lies the problem of rapid access to the nonpyrogenic solution for quality control within the output assembly without risking introduction of endotoxins.

The present invention provides a portable water treatment apparatus for preparation of sterile, nonpyrogenic water using ultrafiltration technology. The apparatus is capable of supplying water for preparation of parenteral solutions for military field hospitals and other emergency field units. The apparatus includes a system for generating chemically pure water in combination with an endotoxin filter output assembly, wherein the endotoxin filter and storage assembly are integral with respect to one another and sealed for prevention of entrance of endotoxins into the storage areas.

In accordance with the present invention, apparatus for generating infusion grade water from feed water comprises a chemical purification assembly in combination with an endotoxin filter output assembly for generating nonpyrogenic water, the chemical purification assembly including a first filter for removing gross particulate and organic impurities from feed water to provide filtered water, reverse osmosis purification means connected to said first filter for generating permeate water from said filtered water, deionization means connected to the reverse osmosis means for deionization of said permeate water, and a second filter for filtering the deionized permeate water to produce chemically pure water; and the said endotoxin filter output assembly including an endotoxin filter adapted to remove from said chemically pure water lipopolysaccharides derived from gram-negative bacteria, for generating non-pyrogenic water, and receiving means integrally sealed with the endotoxin filter for receiving and storing said nonpyrogenic water.

Preferably the said first filter comprises a carbon adsorption filter in the form of a disposable carbon pack having an affinity for free chlorine, chloramines and organics including chlorinated hydrocarbons.

The first filter may be connected to a fines filter having a 5 micron cutoff for protecting the downstream components from fine particulate material emanating from the first filter.

Preferably the reverse osmosis purification means comprises membrane cartridges which are plumbed in parallel and are connected to the said first filter, each of the cartridges comprising spiral wound modules having membranes capable of resisting attack by chlorine, and operable in a pH range between 3 and 11.

The deionization means may comprise a mixed bed deionization chamber.

In a preferred embodiment the said second filter for filtering the deionized permeate water includes a 0.22 micron electronic grade membrane filter.

The apparatus desirably includes a recirculation system including a valve and conduit for controllably recirculating retenate back to the reverse osmosis purification means.

Preferred embodiments of the invention include a temperature compensated conductivity cell in combination with a resistivity monitor for determining the conductivity of said chemically pure water delivered by the said second filter. The monitor desirably includes circuitry for sounding an alarm if the quality of the water fails to meet predefined levels of conductivity, and a three-way electronic valve controlled by the monitor for diverting water from the endotoxin filter output assembly to a drain.

The endotoxin filter is conveniently a polyacrylonitrile hollow-fiber hemofilter, although it will be understood that any ultra-filter having a suitable pore size for removing endotoxins may be employed.

The receiving means preferably includes a manifold having a plurality of tees, each tee having a bag for receiving infusion grade water, the manifold being integrally sealed with the endotoxin filter.

In preferred embodiments a pumping means, such as a rotary or reciprocating pump, may be connected to the first filer for enhancing water movement in the apparatus.

The present invention overcomes the constraints inherent in using distillation and provides a portable unit for generating non-pyrogenic water capable of use in the preparation of parenteral solutions.

The present invention further provides a method for preparing infusion grade water, comprising the steps of chemically purifying a potable feed water by adsorbing gross particles and organic impurities from the feed water to produce filtered water, purifying the filtered water by reverse osmosis to generate a permeate water, deionizing the permeate water, microfiltering the deionized permeate water to produce a chemically pure water; and retentive ultrafiltering the chemically pure water in a retentive ultrafiltering system for the removal of endotoxins to generate infusion grade water, and storing the infusion grade water in a receiving system integrally sealed with the retentive ultrafiltering system.

Other advantages and features of the present invention will become apparent from the following detailed description when taken in conjunction with the drawings in which:

Figure 1 is a block diagram of a chemical water purification unit in accordance with the present invention; and

Figure 2 is a block diagram of an endotoxin filter output assembly in accordance with the present invention.

The present invention provides, in one aspect, an apparatus for preparation of infusion grade water. This infusion grade water may be used in preparation of parenteral solutions. In the preparation of parenteral solutions the starting water must be of a high chemical purity and free of bacteria and pyrogens. The apparatus of the present invention meets these criteria in a two-component system. The first component of the system is a chemical purification unit. Chemical purity is achieved by treating the feed water, i.e. any potable water source, using a sequence of carbon adsorption reverse osmosis, deionization, and membrane filtration.

The chemically pure water from this unit is used to feed a low-molecular-weight cut-off membrane ultrafilter which is an integral part of a presterilized nonpyrogenic manifold with receiving bags. The receiving bags may contain premeasured solutes in sterile nonpyrogenic powder form or concentrated solution form such as electrolytes, carbohydrates, and drugs so that dilution by the nonpyrogenic water will yield typical parenteral solutions ready for administration. As a means of quality assurance, the water purification unit monitors the product water conductivity. If the product water does not conform to a predetermined conductivity level, an alarm system and bypass circuit allows for automatic shutoff.

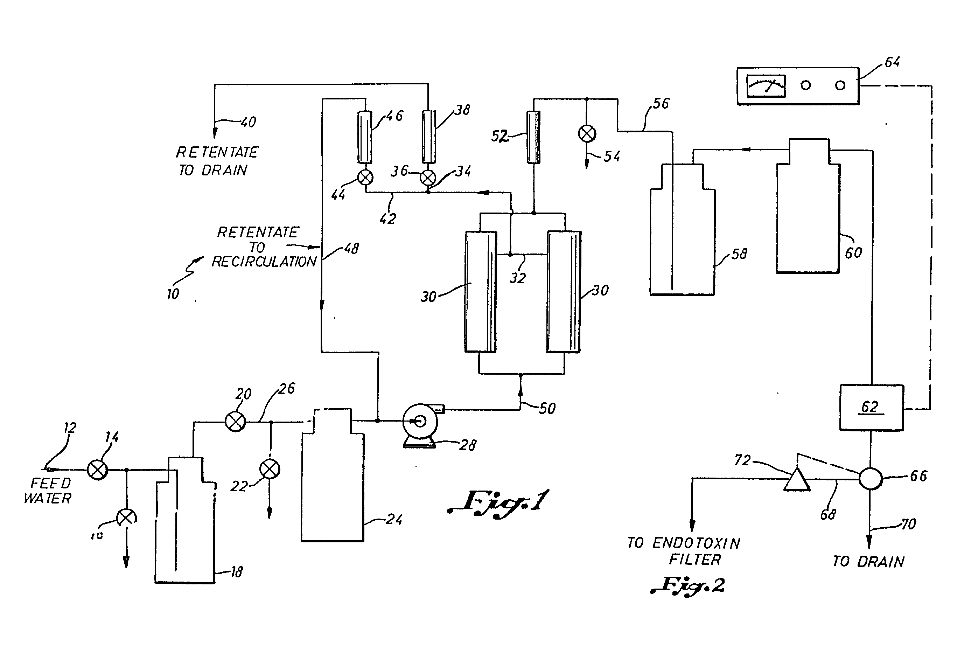

Referring now to the drawings and in particular to Figure 1 where a chemical purification unit 10 in accordance with the present invention is illustrated. In the chemical purification unit 10, feed water is delivered into the system by way of an inlet port 12. This feed water may be monitored by a valve 14 and sampled at a valve port 16.

The feed water may be any potable water, for example from a garden hose. The feed water is first filtered in a carbon adsorption filter 18. This carbon adsorption filter 18 may be a disposable carbon pack. The carbon in this filter 18 is specifically designed to have a high affinity for free chlorine and chloramines as well as a wide variety of organics including chlorinated hydrocarbons. Chloramines have been clearly implicated in hemolytic episodes during hemodialysis and its absence is necessary for any water source to be used for parenteral solution use.

The purification unit 10 is provided with a valve 20 for regulating the flow to the filtered water and a valve sample port 22 for sampling the carbon filtered water.

Since carbon is particularly prone to bacterial contamination because of its porosity and affinity for organics, a disposable filter element may be used so that such bacterial contamination can be avoided by replacement of the filter after each use.

Following the carbon filter unit 18, is a "fines" filter 24. The fines filter 24 may be a 5 micron depth filter used to protect the system from fine particulates which may be released from the carbon in the carbon filter 18. The fine filter 24 is fluidly connected to the carbon filter 18 by way of conduit 26.

Further provided in the chemical purification unit 10 is a pumping apparatus 28 for enhancing the movement of the water throughout the system. The pumping apparatus 28 may be a rotary or reciprocating pump or equivalent having a capacity of 70 gallons (265 litres) per hour and delivering water at 200 psi (1380 kPa) or greater.

The pressurized water is delivered to a plurality of reverse osmosis purification modules or cartridges 30. The RO modules 30 in the preferred embodiment are plumbed in parallel. Each module may be a Film Tech type FT-30 spiral wound module with each module having 6 square feet (0.56 m²) of surface area located in a housing. The FT-30 modules use a membrane that resists attack by chlorine and can be operated at a wide range of pH values on the order of 3 to 11. The retentate stream 42 emanating from the output port 32 of each of the reverse osmosis modules 30 is split. The first leg 34 is provided to deliver some rejected water through a valve 36 and rotameter 38 to a retentate drain 40. The second leg of the retentate stream 42 is provided with a valve 44 and rotameter 46 for recirculating the remainder of the rejected water through conduit 48 and back to the input of the reverse osmosis module 50. Valves 36 and 44 may be of the needle valve type and operate in conjunction with the rotameters for adjusting the flow rates of each stream while maintaining constant feed line pressure at 200 psi (1380 kPa). This allows a variable water recovery so that the unit can be operated at high recovery when the source water is in short supply or low recovery (higher rejection) if the source water is high in salt content.

Permeate flow rate from the RO modules 30 is measured by a third rotameter 52. A valve sample port 54 is provided to monitor the reverse osmosis purification rejection.

Besides removal of large molecule organic impurities the RO modules remove inorganic impurities such as residual electrolytes. This removal assists the deionization unit in removal electrolytes.

The permeate water is then delivered through a fluid conduit 56 to be treated by a mixed bed deionization chamber 58. The mixed bed deionizer 58 removes the residual electrolyte in the permeate water to acceptable concentrations at a level of parts per million.

The deionized permeate solution is then delivered to a microporous 5 micron filter 60 for removal of ion exchange resin fines.

In the preferred embodiment, the rotary or reciprocating pump 28 is a Procon products, V-band clamp mount pump, model CO-1500. The reverse osmosis purification units are FT-30 Film-Tech units manufactured by Film-Tech Corporation, Minnetonka, Minnesota; and the deionizer is of the type manufactured by Cole-Parmer, Chicago, Illinois.

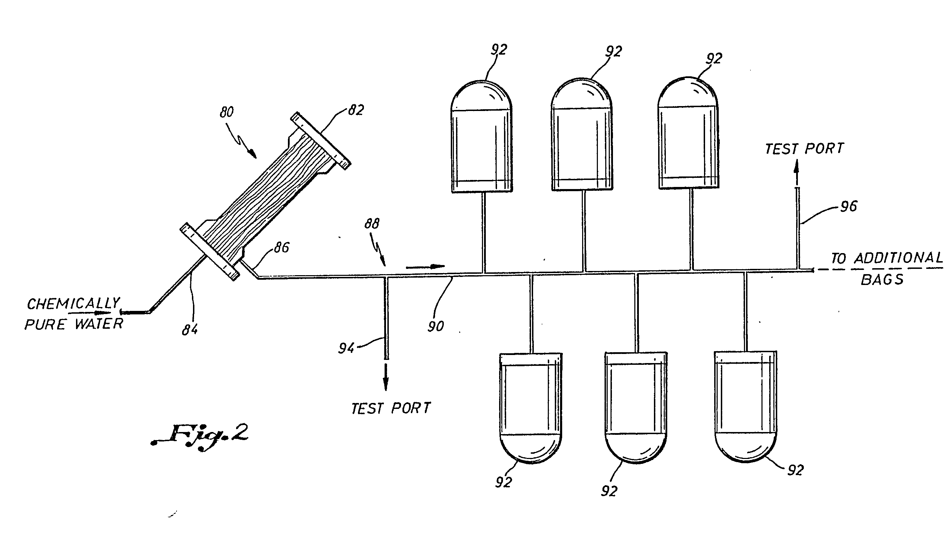

The water delivered from the microporous filter 60 is chemically pure and ultimately delivered to an endotoxin filter output assembly as shown in Figure 2. Prior to delivery of the chemically pure water to the endotoxin filter assembly, its conductivity is determined. Conductivity of the water product is monitored continuously by a 0.01 CM⁻¹ temperature compensated conductivity cell 62, electrically connected to a 0 to 20 megohm-cm resistivity monitor 64. The conductivity monitor 64 may be of the type made by Balsbaugh, model 920M. The monitor 64 provides circuitry for alarm and control valves. The alarm of monitor 64 may be set to sound if the water quality fails to meet a predetermined standard. An electronic three-way valve 66 is provided in the product line and controlled by the resistivity monitor 64. When actuated, the three-way valve 66 diverts the water from the collection assembly line 68 to a drain 70. A pressure sensitive switch 72 is also provided in the collection assembly line to protect the endotoxin filter shown in Figure 2 from excessive pressure.

The apparatus for preparation of infusion grade water includes the chemical purification unit 10 as described above in conjunction with an endotoxin filter assembly 80 as shown in the drawing of Figure 2. In the filter assembly 80, a bacterial and endotoxin retentive membrane filter 82 is provided. The filter 82 receives at its input port 84 chemically pure water from the chemical purification unit 10 shown in Figure 1 and described above. The chemically pure water passes over the membranes making up the endotoxin filter 82. The endotoxin filter used in the preferred embodiment is made by Asahi Medical Company Ltd. It is a polyacrylonitrile hollow-fiber hemofilter. The filter has a 1.4 m² surface area and is presterilized by ethylene oxide. The solute molecular weight cutoff of this filter is estimated at 10—15,000 Daltons. The lipopolysaccharides derived from the cell wall of negative bacteria are polymeric materials having aggregate molecular weights between 400,000 and 4,000,000 Daltons. Thus, the low molecular weight endotoxin filter 82 utilized in the endotoxin filter assembly 80 will trap lipopolysaccharides and retain them within the filter itself.

The filtrate delivered from the output 86 of the filter 82 is delivered to a presterilized non-pyrogenic manifold and receiving bag assembly 88. The assembly 88 is integrally sealed with the filter 82. The manifold 90 may be fabricated from 3/16" (5 mm) I.D. medical grade Tygon tubing and sterilizable plastic, for example polypropylene, tees (not shown). A plurality of receiving bags 92 such as blood bags for example are connected to the manifold 90 via the polypropylene tees. Typically, 6 to 8 bags are used on each manifold assembly although a greater number may be utilized.

The membrane filter 82 is sealed to the sterile bags 92 via the manifold system 90. Entrance to the receiving bags 92 can only be through the retentive membrane filter 82. Thus, sterile, non-pyrogen solutions can be prepared using the chemically pure water generated by the chemical purification unit 10.

An additional tee 94 may be placed at the head of the manifold 90 for sampling the filtrate from the filter 82 for presence of endotoxins. This tee would contain a sterile septum and cap.

As shown in Figure 2, additional bags may be placed along the manifold assembly 90 with additional endotoxin test sample ports such as port 96.

Operationally, the apparatus of the present invention for preparation of infusion grade water for use in preparation of parenteral solutions includes the preparation of a chemically pure water in conjunction with a retentive ultrafiltration process. The preparation of the chemically pure water in the preferred embodiment assumes obtaining potable water and performing a sequence of carbon adsorption, reverse osmosis purification, deionization and microporous filtration to obtain a chemically pure solution.

In the preferred embodiment, retentive ultrafiltration of the chemically pure water is achieved through a filter having a low molecular weight cutoff membrane. The filtrate from the ultrafiltration step is delivered into receiving bags by way of a presterilized nonpyrogenic manifold integrally sealed to the low molecular weight filter.

An additional step in the preparation of infusion grade water involves the sampling of the filtrate from the ultrafiltration process and testing for the presence of endotoxins. This testing may be performed by either the historical rabbit test method, or the *in vitro* limulus amebocyte lysate (LAL) test. This quality control measure is used to validate sterility and guarantee quality of the product.

The infusion grade water may be utilized in the preparation of parenteral solutions. In the process of preparing parenteral solutions using the apparatus of the present invention, the receiving bags 92 illustrated in Figure 2 may contain premeasured solutes in sterile non-pyrogenic powder or concentrated solution form, such as electrolytes, carbohydrates, or drugs. Dilution by nonpyrogenic water will then yield typical intravenous solutions ready for administration.

While the present invention has been described and illustrated with respect to a preferred embodiment, it will be understood to those skilled in the art that various modifications and changes such as the substitution of crosses for tees in the manifold, are contemplated to be within the spirit and scope of the invention as set forth in the appended claims.

**Claims**

1. Apparatus for generating infusion grade water from feed water, comprising a chemical purification assembly in combination with an endotoxiin filter output assembly for generating nonpyrogenic water, the chemical purification assembly including a first filter for removing gross particulate and organic impurities from feed water to provide filtered water, reverse osmosis purification means connected to said first filter for generating permeate water from said filtered water, deionization means connected to the reverse osmosis means for deionization of said permeate water, and a second filter for filtering the deionized permeate water to produce chemically pure water; and the said endotoxin filter output assembly including an endotoxin filter adapted to remove from said chemically pure water lipopolysaccharides derived from gram-negative bacteria, for generating non-pyrogenic water, and receiving means integrally sealed with the endotoxin filter for receiving and storing said nonpyrogenic water.

2. Apparatus according to claim 1, in which the said first filter comprises a carbon adsorption filter in the form of a disposable carbon pack having an affinity for free chlorine, chloramines and organics including chlorinated hydrocarbons.

3. Apparatus according to claim 1 or claim 2, further comprising a fines filter having a 5 micron cutoff, connected to the first filter, for protecting the downstream components from fine particulate material emanating from the first filter.

4. Apparatus according to any one of claims 1 to 3, in which the reverse osmosis purification means comprises membrane cartridges which are plumbed in parallel and are connected to the said first filter, each of the cartridges comprising spiral wound modules having membranes capable of resisting attack by chlorine, and operable in a pH range between 3 and 11.

5. Apparatus according to any one of claims 1 to 4, further comprising a recirculation system including a valve and conduit for controllably recirculating retentate back to the reverse osmosis purification means.

6. Apparatus according to any one of claims 1 to 5, further comprising a temperature compensated conductivity cell in combination with a resistivity monitor for determining the conductivity of said chemically pure water delivered by the said second filter.

7. Apparatus according to claim 6, in which the said monitor includes circuitry for sounding an alarm if the quality of the water fails to meet predefined levels of conductivity, and a three-way electronic valve controlled by the monitor for diverting water from the endotoxin filter output assembly to a drain.

8. Apparatus according to any one of claims 1 to 7, in which the endotoxin filter is a polyacrylonitrile hollow-fiber hemofilter.

9. Apparatus according to any one of claims 1 to 8, in which the said receiving means includes a manifold having a plurality of tees, each tee having a bag for receiving infusion grade water, the manifold being integrally sealed with the endotoxin filter.

10. A method of preparing infusion grade water, comprising the steps of chemically purifying a potable feed water by adsorbing gross particles and organic impurities from the feed water to produce filtered water, purifying the filtered water by reverse osmosis to generate a permeate water, deionizing the permeate water, microfiltering the deionized permeate water to produce a chemically pure water; and retentive ultrafiltering the chemically pure water in a retentive ultrafiltering system for the removal of endotoxins to generate infusion grade water, and storing the infusion grade water in a receiving system integrally sealed with the retentive ultrafiltering system.

**Patentansprüche**

1. Vorrichtung zur Herstellung von Wasser mit Infusionsqualität aus Speisewasser, mit einer chemischen Reinigungseinheit kombiniert mit einer Endotoxinfilter-Ausgabe-Einheit zur Herstellung von nicht-pyrogenem Wasser, wobei die chemische Reinigungseinheit folgende Untereinheiten aufweist: ein erster Filter zur Entfernung grober Teilchen und organischer Verunreinigungen aus dem Speisewasser, der filtriertes Wasser liefert, eine Umkehrosmose-Reinigungsanlage, die mit besagtem ersten Filter verbunden ist und aus besagtem filtrierten Wasser Permeatwasser erzeugt, eine Entionisierungs-Anlage, die mit der Umkehrosmose-Anlage verbunden ist und die besagtes Permeatwasser entionisiert und ein zweiter Filter, der das entionisierte Permeatwasser filtriert und chemisch reines Wasser liefert; und die Endotoxinfilter-Ausgabe-Einheit folgende Untereinheiten aufweist: Ein Endotoxinfilter, der geeignet ist, aus besagtem chemisch reinen Wasser Lipopolysaccharide, die von gramnegativen Bakterien stammen, zu entfernen und eine Aufnahmeanlage, die direkt mit dem Endotoxinfilter dicht verbunden ist und die besagtes nicht-pyrogenes Wasser aufnimmt und speichert.

2. Vorrichtung nach Anspruch 1, wobei der besagte erste Filter einen Kohle-Adsorptionsfilter in Form eines wegwerfbaren Kohleeinsatzes enthält, der freies Chlor, Chloramine und organische verbindungen einschließlich chlorierter Kohlenwasserstoffe bindet.

3. Vorrichtung nach Anspruch 1 oder Anspruch 2, die weiterhin einen Feinfilter mit einer Trennfähigkeit von 5 µ enthält, der mit dem ersten Filter verbunden ist und der die in Fließrichtung folgenden Anlagenteile vor feinem Teilchenmaterial schützt, welches aus dem ersten Filter stammt.

4. Vorrichtung nach einem der Ansprüche 1 bis 3, wobei die Umkehrosmose-Reinigungsanlage Membranpatronen enthält, welche parallel eingebaut und mit dem besagten ersten Filter verbunden sind, wobei jede Patrone einen spiralförmigen Einsatz mit Membranen enthält, die beständig gegen Chlor und einsetzbar in einem pH-Bereich zwischen 3 und 11 sind.

5. Vorrichtung nach einem der Ansprüche 1 bis 4, die weiterhin eine Rückführungssystem mit einem Ventil und Rohrleitungen zur kontrollierten Rückführung des Retentats zur Umkehrosmose-Reinigungsanlage enthält.

6. Vorrichtung nach einem der Ansprüche 1 bis 5, die weiterhin eine temperaturkompensierte Leitfähigkeitsmeßzelle in Verbindung mit einem Widerstandsmeßgerät enthält, um die Leitfähigkeit des besagten chemische reinen Wassers, welches der besagte zweite Filter liefert, zu bestimmen.

7. Vorrichtung nach Anspruch 6, wobei das besagte Meßgerät eine Schaltung, welche Alarm gibt, falls die Wasserqualität vorbestimmte Grenzwert für die Leitfähigkeit überschreitet, und ein elektronisches Dreiwege-Ventil einschließt, das vom Meßgerät gesteuert wird und Wasser vor der Endotoxinfilter-Ausgabe-Einheit in einem Abfluß umleiten kann.

8. Vorrichtung nach einem der Ansprüche 1 bis 7, wobei der Endotoxinfilter eine Hemofilter aus Polyacrylnitril-Hohlfasern ist.

9. Vorrichtung nach einem der Ansprüche 1 bis 8, wobei die besagte Aufnahmeanlage einen Verteiler mit mehreren T-Stücken einschließt, der direkt mit dem Endotoxinfilter dicht verbindunden ist, wobei sich an jedem T-Stück ein Behälter zur aufnahme des Wassers mit Infusionsqualität befindet.

10. Verfahren zur Herstellung von Wasser mit Infusionsqualität, das folgende Schritte aufweist: chemische Reinigung des trinkbaren Spreisewassers durch Adsorption grober Teilchen und organischer Verunreinigungen aus dem Speisewasser, um filtriertes Wasser herzustellen, Reinigung des filtrierten Wassers durch Umkehrosmose, um Permeatwasser zu erzeugen, Entionisierung des Permeatwassers, Mikrofiltration des entionisierten Permeatwassers um chemisch reines Wasser herzustellen; und rückhaltefähige Ultrafiltration des chemisch reinen Wassers in einem rüchhaltefähigen Ultrafiltrationssystem zur Entfernung von Endotoxinen, um Wasser mit Infusionsqualität zu erzeugen, und Speicherung des Wassers mit Infusionsqualität in einem Aufnahmesystem, welches direkt mit dem rückhaltefähigen Ultrafiltrationssystem dicht verbunden ist.

**Revendications**

1. Dispositif pour produire une eau utilisable pour perfusions à partir d'une eau d'alimentation, comprenant un ensemble de purification chimique en combinaison avec un ensemble de sortie de filtres à endotoxines pour produire une eau non pyrogène, l'ensemble de purification chimique comprenant un premier filtre pour éliminer les particules grossières et les impuretés organiques d'une eau d'alimentation pour fournir une eau préfiltrée, un moyen de purification par osmose inverse relié audit premier filtre pour produire une eau filtrée à partir de ladite eau préfiltrée, un moyen de désionisation relié au moyen d'osmose inverse pour la désionisation de ladite eau filtrée, et un second filtre pour filtrer l'eau filtrée désionisée pour produire une eau chimiquement pure; et ledit ensemble de sortie de filtres à endotoxines comprenant un filtre à endotoxines adapté pour éliminer de ladite eau chimiquement pure les lipopolysaccharides provenant de bactéries gram-négatives, pour produire une eau non pyrogène, et des moyens récepteurs fermés complètement avec le filtre à endotoxines pour recevoir et conserver ladite eau non pyrogène.

2. Dispositif selon la revendication 1, dans lequel ledit premier filtre comprend un filtre à adsorption en charbon sous la forme d'un garnissage de charbon disponible ayant une affinité pour le chlore libre, les chloramines et les produits organiques comprenant des hydrocarbures chlorés.

3. Dispositif selon la revendication 1 ou la revendication 2, comprenant de plus un filtre à fines ayant un pouvoir séparateur de 5 microns, relié au premier filtre, pour protéger les composants en aval de la matière à fines particules provenant du premier filtre.

4. Dispositif selon l'une quelconque des revendications 1 à 3, dans lequel le moyen de purification par osmose inverse comprend des cartouches à membrane qui sont disposées verticalement en parallèle et sont reliées audit premier filtre, chaque cartouche comprenant des modules enroulés en spirales ayant des membranes capables de résister à l'attaque par le chlore, et qui peut fonctionner dans une gamme de pH entre 3 et 11.

5. Dispositif selon l'une quelconque des revendications 1 à 4, comprenant de plus un système de recirculation comprenant une vanne et une conduite pour faire recirculer de manière contrôlable le produit retenu vers le moyen de purification par osmose inverse.

6. Dispositif selon l'une quelconque des revendications 1 à 5, comprenant de plus une cellule de conductivité compensée en température en combinaison avec un appareil de contrôle de résistivité pour déterminer la conductivité de ladite eau chimiquement pure délivrée par ledit second filtre.

7. Dispositif selon la revendication 6, dans lequel ledit appareil de contrôle comprend un circuit pour actionner une alarme si la qualité de l'eau ne correspond pas à des niveaux de conductivité préalablement définis, et une vanne électronique à trois voies commandée par l'appareil de contrôle pour dévier l'eau de l'ensemble de sortie de filtres à endotoxines vers une canalisation d'évacuation.

8. Dispositif selon l'une quelconque des revendications 1 à 7, dans lequel le filtre à endotoxines est un hémofiltre à fibres creuses de polyacrylonitrile.

9. Dispositif selon l'une quelconque des revendications 1 à 8, dans lequel ledit moyen récepteur comprend un collecteur possédant une pluralité de tés, chaque té possédant une poche pour recevoir une eau utilisable pour perfusions, le collecteur étant fermé intégralement avec le filtre à endotoxines.

10. Procédé de préparation d'une eau pour perfusions, comprenant les étapes consistant à purifier chimiquement une eau d'alimentation potable en adsorbant les particules grossières et les impuretés organiques de l'eau d'alimentation pour produire une eau préfiltrée, à purifier l'eau préfiltrée par osmose inverse pour produire une eau filtrée, à désioniser l'eau filtrée, à microfiltrer l'eau filtrée désionisée pour produire une eau chimiquement pure; et à faire une ultrafiltration de rétention de l'eau chimiquement pure dans un système d'ultrafiltration rétentive pour l'élimination des endotoxines pour produire une eau utilisable pour perfusions, et à conserver l'eau utilisable pour perfusions dans un système récepteur fermé complètement avec le système d'ultrafiltration rétentive.

RETENTATE
TO DRAIN

RETENTATE
TO
RECIRCULATION

FEED
WATER

_Fig.1_

TO ENDOTOXIN
FILTER

TO DRAIN

_Fig.2_

0 056 855

Fig.2